# EUROPEAN PATENT APPLICATION

(11) **EP 4 417 682 A1**
(43) Date of publication of application: **21.08.2024**
(21) Application number: 23156835.3
(22) Date of filing: 15.02.2023
(51) Int. Cl.: C12M 1/00, C12M 1/06, C12M 1/34

(54) **FLOW CONTROL TRAY FOR ALGAE CULTIVATION WITH ELEVATIONS**

(71) Applicant: Ad Astra ehf, 201 Kopavogur (IS)
(72) Inventor: Podola, Björn, 50969 Cologne (DE); Mayer, Roy, CA 2628 Delft (NL)
(74) Representative: dompatent von Kreisler Selting Werner - Partnerschaft von Patent- und Rechtsanwälten mbB

(57) **Abstract**

Flow control tray for an algae cultivation fluid comprising a ground layer extending in a ground plane from a first end to a second end and a plurality of elevations extending from the ground layer in a first direction, wherein the ground layer is adapted to guide a flow of an algae cultivation liquid above the ground plane from the first end to the second end to form a general flow direction in parallel to the ground layer, wherein each of the plurality of elevations comprises a support distanced in a direction orthogonally to the ground plane and adapted to support an algae cultivation membrane, and wherein each of the plurality of elevations has a three-dimensional shape preferably adapted to divert the cultivation fluid from the general flow direction.

## Description

The current invention concerns a flow control tray for an algae cultivation fluid, a system for algae cultivation comprising a flow control tray as well as an algae cultivation layer and the usage of a system for algae cultivation.

It is already known from the state of the art to produce algae such as microalgae, for example *Haematococcus pluvialis,* containing the carotenoid Astaxanthin by usage of Porous Substrate Bioreactors (PSBR). In PSBRs algae are cultivated in biofilms on humid surfaces with a very low water content of the culture.

According to the currently known state of the art, PSBRs are used in combination with off-shelf greenhouse equipment wherein trays are used to control the distribution of a cultivation fluid such as a nutrient medium in a greenhouse bench system.

According to the state-of-the-art microalgae and, for example, Astaxanthin-containing microalgae such as *Haematococcus* are produced on an upper cultivation membrane also known as microalgae cultivation layer of the PSBRs wherein the membrane is permeable for water and nutrients such as the algae cultivation fluid. On the other hand, the membrane prevents the algae to suspend in the bulk of the aqueous algae cultivation fluid formed by water and nutrients (=nutrient solution). The algae cultivation fluid is supplied from the bottom side of the membrane by a continuous circulation of the cultivation liquid along the bottom surface of the membrane. Such a continuous cultivation liquid circulation can be, for example, established in a standard greenhouse bench wherein the greenhouse bench comprises a liquid tub wherein on an upper end, the cultivation fluid is supplied to the tub and drained at a lower end of the tub.

According to the state of the art, the production setting of the microalgae cultivation will make use of an inclined standard greenhouse bench system, wherein vacuum-formed plastic trays are used in combination with the inclined greenhouse bench system to distribute the water and nutrients. In order to cultivate microalgae, in standard, greenhouse benches a hydrophilic sheet-like algae cultivation layer is provided. Deviating from the above-described standard, greenhouse bench systems of other types of inclined benches can be used which are suitable to distribute water and nutrients.

The microalgae cultivation layer according to the state of the art is formed by a sheet-like composite layer, consisting of at least two functional units, wherein a thin and fine-porous membrane functioning as an upper microalgae cultivation layer and at least one lower distribution layer consisting for example of a voluminous polyester fibre nonwoven distributes the nutrient medium from the underlying water nutrient supply structures to the membrane. According to the state of the art, it is also known to use paper- sheets, carpets, gauzes, glass fibres and/or sponges for the cultivation of microalgae in the PSBR.

The algae cultivation method and systems according to the aforementioned state of the art have the disadvantages that:
1. To support the fine-porous membrane, the distribution layer is obligatory. This increases complexity of production and material demand of the sheet-like composite layer.
2. According to the state of the art, it is known to use as a distribution layer: paper- sheets, carpets, gauzes, glass fibres and/or sponges for the cultivation of microalgae in the PSBR. The aforementioned materials have in common the disadvantage that they provide a small/limited open space in the distribution layer, generating a high resistance for the flow of the supplied algae cultivation fluid. Thereby, only a limited amount of the cultivation fluid per time interval can be applied to the bioreactor (below 15 L m⁻¹ h⁻¹, in particular below 5 L m⁻¹ h⁻¹), wherein the growth rate of the microalgae is limited by the supplied amount of fresh cultivation fluid and thereby dependent on the flow rate of the cultivation fluid. This occurs in particular in situations when a long flow path in long benches/trays is used and could limit productivity of the cultivation gradually along the flow direction.
3. In addition, low flow rates and narrow spaces are supporting biofouling in the distribution layer, leading to clogging of the layer's structure, resulting in disturbance of flow. This effect can partially or fully disrupt the cultivation process.
4. Further, during the cultivation process, gas released from cultivation medium or by algae photosynthesis accumulates between the fine-porous membrane and a distribution layer. By this, the membrane carrying microalgae detaches from the distribution layer. In consequence, microalgae are cut off from water and nutrient supply by a gas layer, damaging the culture in the detached regions, in particular due to drying out.
5. In some settings, there might be laminar flow connected to unfavourable mixing of cultivation liquid, reducing mass transfer via the membrane.

It is the task of the current invention to solve the aforementioned problems and disadvantages of the known state of the art for algae such as microalgae cultivation methods and systems. Specifically, the invention intends to provide an improved method and systems which enable a faster microalgae growth and more stable process with at the same time simplified structures and reduction of used components.

The aforementioned tasks are solved by the independent claims of the current invention.

According to a first aspect of the current invention, a flow control tray for algae cultivation such as microalgae cultivation is provided. The flow control tray according to the current invention comprises a ground layer extending in a ground plane from a first end to a second end and a plurality of elevations extending from the ground layer in a first direction. The ground layer is adapted to guide a flow of an algae cultivation fluid such as a nutrient medium above the ground plane from the first end to the second end to form a general flow of the cultivation fluid in a direction in parallel to and above the ground layer.

The flow of the algae cultivation above the ground layer can be a continuous stream of the cultivation fluid. Alternatively, according to the present invention, the algae cultivation fluid can be also supplied in a discontinuous stream, wherein the cultivation fluid is supplied in time intervals to the flow control tray.

It can be provided that the ground plane is arranged in a plane inclined to the horizontal plane, so that the first end of the ground layer of the flow control tray is arranged higher than the second end. The horizontal plane is defined to extend in a plane orthogonal to the vector of the gravity. The inclined arrangement of the ground layer of the flow control layer has the effect that the cultivation fluid which is supplied in the area of the first end of the flow control tray forms under the gravity force a fluid flow with a general flow direction to the second end of the control tray above the ground layer of the flow control tray.

The ground plane according to the invention can thereby be formed with different thickness along the general flow direction to provide an inclined ground plane.

The ground layer can have two transversal sides facing each other formed by the first end and the second end and two longitudinal sides facing each other and extending in the direction of the general flow direction. An area of the ground layer can be defined as area enclosed between the transversal and longitudinal sides. The ground layer can have a rectangular or quadratic shape.

Each of the plurality of elevations comprises a support distanced in a direction orthogonally to the ground plane and adapted to support an algae cultivation membrane. Each of the plurality of elevations is provided with a three-dimensional geometry/shape. The geometry/shape may be adapted to divert the cultivation fluid from the general flow direction.

Optionally, each of the plurality of elevations has a three-dimensional shape to induce a laminar vortex with a horizontal axis of rotation in the cultivation fluid.

Preferably, each support of the plurality of the elevations is arranged in a second supporting plane which extends parallel to the ground plane and is distanced to the ground plane by a support height.

Alternatively, according to the invention, it can be also provided that the plurality of elevations is arranged in at least two different supporting planes, wherein the supports are arranged distanced to the ground plane by at least two different support heights above the ground plane.

Preferably, the area of the second supporting plane is as small as possible to allow optimal transfer of cultivation liquid through the cultivation membrane.

The plurality of elevations can have a three-dimensional shape selected as one or more elements of the list of: a cylinder, a rectangle, a pyramid or a cone, a half-sphere, a half-droplet. The three-dimensional shape can be inclined relative to the ground plane.

Optionally, each of the elevations is formed as a ramp which extends from the ground plane in the first direction to the upper support in a first support height. The ramp can for example have a rectangular, quadratic, trapezoidal or round shape.

The support for the membrane by each of the elevation may be formed by either an upper support surface or an upper support edge, or alternatively by an upper support point.

The elevations may be preferably formed as a triangular ramp extending in the direction of the general flow direction from a triangular base arranged in the ground plane to the support distanced in the first direction to the ground plane, wherein a width of the triangular ramp narrows in the general flow direction.

Optionally, the triangular ramps are provided with a triangular tip as support or wherein the triangular ramps are formed as a truncated triangular with a triangular edge as support. Alternatively, the triangular ramps can be also provided with an upper support face.

The elevations can be evenly distributed over an area of the ground layer. Alternatively, the elevations can be also randomly/irregularly arranged over an area of the ground layer.

Preferably, the elevations are arranged in a plurality of lines, wherein the lines extend orthogonal to the general flow direction, wherein the lines are evenly distributed along the general flow direction and spaced apart by a row distance.

Additionally, the elevations arranged in a line can be spaced apart with respect to each other in the direction of the line by a clearance distance between neighboured elevations.

Furthermore, according to the current invention, it can be provided that in two consecutive lines of elevations seen in the direction of the general flow, the elevation patterns of the neighboured lines can be offset by a first offset distance. As a result, the elevations of a second line following in the flow direction to a first line, are arranged in the clearance area of the elevations of the first line so that over the entire area of the ground plane in the general flow direction, an alternating pattern of the lines of elevations can be provided.

The aforementioned configuration leads to the effect that the cultivation fluid which flows through a first line of elevations and specifically the cultivation fluid which flows through the clearance areas between the elevations of the first line then moves forward in the general flow direction to a second line with a alternated pattern so that the area of flow which flows through the clearance areas is guided toward the elevations of the second line and is thereby diverged so that by the provided alternating elevations patterns of the consecutive lines of elevations seen in the general flow direction results in a continuously diverging cultivation fluid.

Furthermore, according to the invention, it can be provided that the continuous flow of the algae cultivation fluid forms a continuous liquid film above the ground layer with a fluid layer height in the first direction.

Preferably, the fluid layer height in a direction orthogonally to the ground plane is equal to the support height of the elevations in the same direction.

The plurality of elevations can be spaced apart by minimum distance in the range of 0.1cm to 10.0cm in a direction parallel to the ground plane.

Furthermore, according to the invention, the ground layer and the plurality of elevations can be manufactured by a vacuum-forming process from a continuous material sheet.

According to the invention, the ground layer can be also formed from a single metal sheet, wherein the elevations can be embossed or punched from the metal sheet. The ground layer can be also manufactured by CNC milling, 3D printing, hydroforming, stamping, cutting, or a combination of these manufacturing processes. The elevations can be produced separately by for eample 3D printing or injection moulding and mechanically or chemically joint with the ground plane.

In an alternative configuration of the current invention, the ground layer can be also formed by a plurality of trusses or structures extending in the ground plane between the plurality of elevations and connected to the plurality of elevations to form a mesh-like structure between the plurality of elevations which extends in the ground plane and where there is a different or no material in-between the elevations and the plurality of trusses. The plurality of trusses can also be provided with different thicknesses so to generate an inclination of the ground plane.

Materials for the trays and/or elevations can be plastic and/or metal to meet the requirements of being "waterproof", inert and non-toxic. Non-waterproof and/or reactive materials can also be coated with a suitable coating fulfilling these requirements.

The ground layer can be formed as a continuous ground wall or alternatively can be formed as a perforated plate or as a mesh-like structure.

The ground layer can be provided to further comprise at least one sidewall extending in the first direction to laterally delimit the ground layer and to form a tub. Preferably, the ground layer is provided with sidewalls on each lateral and longitudinal side to form a surrounding wall so that the ground layer forms a tub.

The ground layer can be additionally formed with a plurality of ribs to increase the bending stiffness of the ground layer. The plurality of ribs can be arranged in parallel to the longitudinal sides and/or transversal sides of the ground layer. The plurality of ribs can be formed to extend along the general flow direction.

More preferably, the plurality of ribs can extend in the first direction to an extension height which is lower than the first support height.

The second end of the tray may be modified to optimize the outflow of cultivation liquid into a drain system.

According to a second aspect, the current invention provides a system for algae cultivation which comprises a flow control tray according to the first aspect of the current invention and furthermore comprises an algae cultivation layer.

The algae cultivation layer can be formed from a mineral or hydrophilic organic material or combinations thereof. The algae cultivation layer can be specifically formed from at least one element selected from the list of organic materials: paper, cellulose ester, cellulose acetate, mixed cellulose ester, cellulose, cellulose nitrate, polyamides, polyesters, polyolefins, and/or graphene.

Alternatively, or in addition, the cultivation membrane can comprise at least one inorganic material selected from the list of: porous ceramic material and/or glass fibre.

The algae cultivation layer can be formed by a fluid permeable membrane.

The algae cultivation layer can be formed by a hydrophilic cultivation membrane with a pore size of less than 50 µm. More preferably, the pore size of the cultivation layer ranges from 0.1 µm to 25 µm. In specific examples of the current invention, medium pore sizes of 10 µm, 8.0 µm and 7.5 µm, 5.0 µm, 1.2 µm, 0.4 µm and 0.1 µm were used.

The algae cultivation layer can be furthermore formed by an algae cultivation membrane which can have a thickness of less than 50µm and/or a pore size of less than 25µm and preferably formed by a polybutylene terephthalate.

The system can further comprise a liquid supply unit which is configured to be arranged in the area of the first end of the ground layer and further configured to evenly supply the incoming algae cultivation fluid to the first end of the ground layer. The liquid supply unit can provide the effect to improve the inflow of the cultivation liquid from a supply pipe, such as avoiding generation of air bubbles and spilling.

According to a third aspect, the current invention provides the usage of a system for algae cultivation according to the second aspect of the invention for the cultivation of microalgae, especially for the cultivation of Astaxanthin-containing microalgae.

In the following exemplary embodiments, in accordance with the present invention are described and referenced to the attached figures:
Fig. 1A shows a schematic perspective view of a first exemplary embodiment of a flow control tray;
Fig. 1B shows a second exemplary embodiment of a flow control tray;
Fig. 1C shows a third exemplary embodiment of a flow control tray according to the current invention;
Figures 2A-2C show three different exemplary embodiments of elevations for the flow control tray according to the current invention;
Fig. 3 shows a cut-through view of a further exemplary embodiment of a flow control tray in accordance with the current invention;
Fig. 4 shows a further perspective view of an exemplary flow control tray wherein the tray is formed as a tub, and
Fig. 5 shows an example of an elevation pattern for a flow control tray according to the current invention.

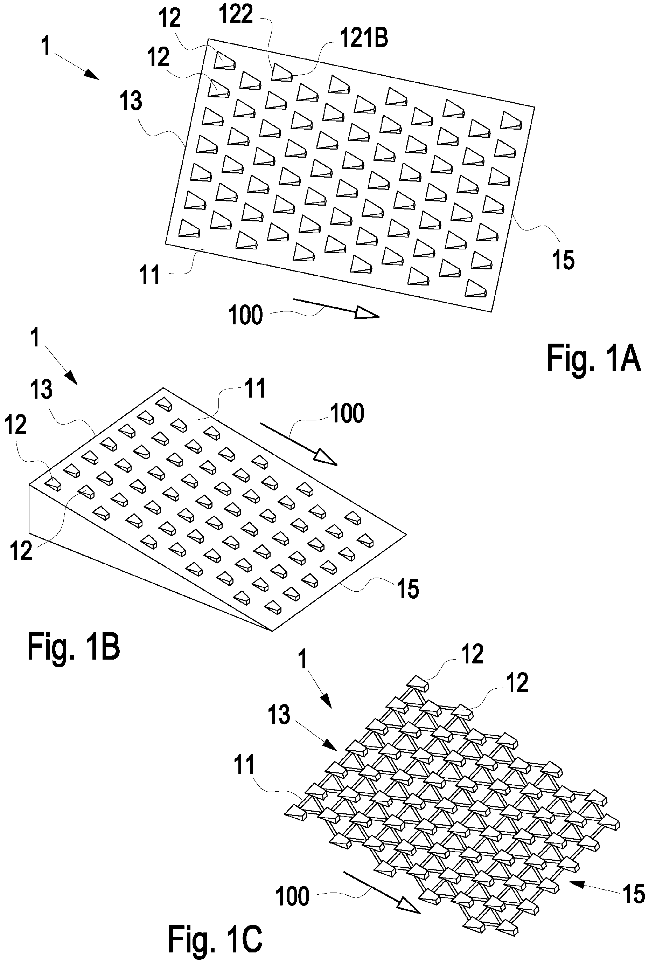

Fig. 1A shows a first exemplary embodiment of a flow control tray 1 according to the current invention in perspective view. The flow control tray 1 for algae cultivation comprises a ground layer 11 which extends in a ground plane from a first end 13 to a second end 15. The ground layer 11 according to Fig. 1A is formed by a continuous flat sheet material from which a plurality of elevations 12 extends from the aforementioned ground layer 11 in a first direction 14.

The ground layer 11 is adapted to guide a flow of an algae cultivation liquid above the ground plane from the first end 13 to the second end 15 to form a general flow direction 100. The general flow direction is shown in Fig. 1A with the arrow 100 in parallel to the ground layer 11. Each of the plurality of elevations 12 comprise a support 121 which is distanced in a direction orthogonally to the ground plane and adapted to support an algae cultivation membrane 2 (not shown in Fig. 1). The support 121 according to Fig. 1A is formed as an upper support edge 121B. As can be taken from Fig. 1A, each of the plurality of elevations 12 are three-dimensional shaped and adapted to divert the cultivation fluid from the general flow direction 100.

Each of the plurality of elevations 12 has a three-dimensional shape to induce a laminar vortex with a horizontal axis of rotation in the cultivation fluid. Specifically, in the exemplary embodiment according to Fig. 1A, each of the elevations 12 is formed as a ramp which extends from the ground plane 11 in a first direction 14 to the upper support 121 in a first support height h. Each of the shown elevations 12 are formed as a triangular ramp which extends in the direction of the general flow direction 100 from a triangular base 122 in the ground plane to the support 121 distanced in the first direction 14 to the ground plane wherein a width of the triangular ramp narrows in the direction of the flow direction 100.

The Fig. 1B shows a second exemplary embodiment of a flow control tray 1 according to the present invention. The exemplary embodiment according to Fig. 1B differs from the configuration of Fig. 1A in that the ground layer 11 which extends in a ground plane from a first end 13 to a second end 15 is formed by a wedge-shaped part itself wherein the ground layer 11 is inclined with respect to a supporting plane wherein the inclination causes the first end 13 to have a greater distance to the supporting plane than the second end 15. The aforementioned inclination causes a cultivation fluid which is disposed to the flow control tray 1 in the area of the first end 13 to flow under the existing gravitational force to the second end 15 of the flow control tray 1.

Fig. 1C shows a further third exemplary embodiment of the flow control tray 1, wherein the embodiment differs from the embodiments of Fig. 1A and Fig. 1B in the configuration of the ground layer 11. Specifically, the ground layer 11 of the flow control tray 1 according to Fig. 1C is formed by a plurality of connection parts/trusses which connect the plurality of elevations 12 to each other. Thereby, the flow control tray 1 according to Fig. 1C, specifically the ground layer 11, is formed as a mesh-like structure, at the intersection of the mesh-like structure, an elevation 12 is arranged to form the flow control tray 1.

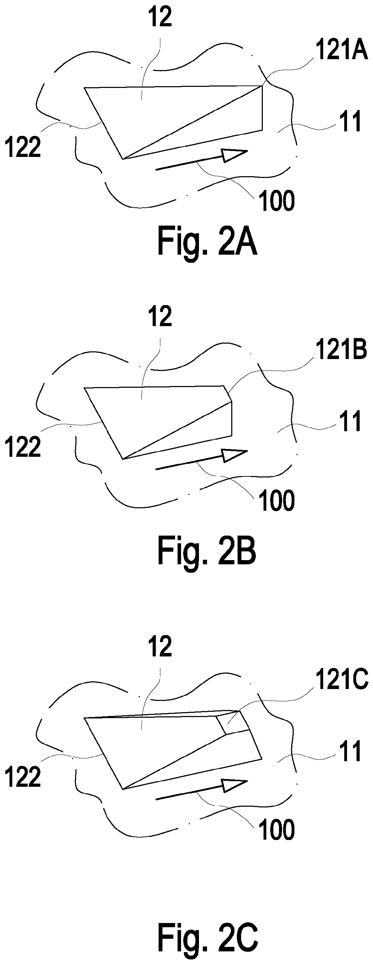

The Figures 2A through 2C show three different exemplary embodiments of the elevations 12 for the flow control tray 1 wherein each of the elevations 12 in Figures 2 are formed as a ramp which extends from the ground plane 11 in a first direction to an upper support 121 in a first support height h. The configurations according to Figures 2 of the elevations 12 differ in the configuration of the upper support 121. Specifically, Fig. 2A shows the configuration wherein an upper support point 121A is formed. The elevation 12 according to Fig. 1A is formed as a triangular ramp which extends in the direction of the general flow direction 100 from a triangular base 122 in the ground plane to the support 121 distanced in the first direction 14 to the ground plane wherein the triangular ramp narrows in the general flow direction and wherein according to Fig. 2A, the triangular ramp is provided with a triangular tip 121A as support 121. In Fig. 2B, the triangular ramp is formed as a truncated triangular with a triangular edge 121B as upper support 121. In Fig. 2C, the ramp is formed as an upper support surface 121C.

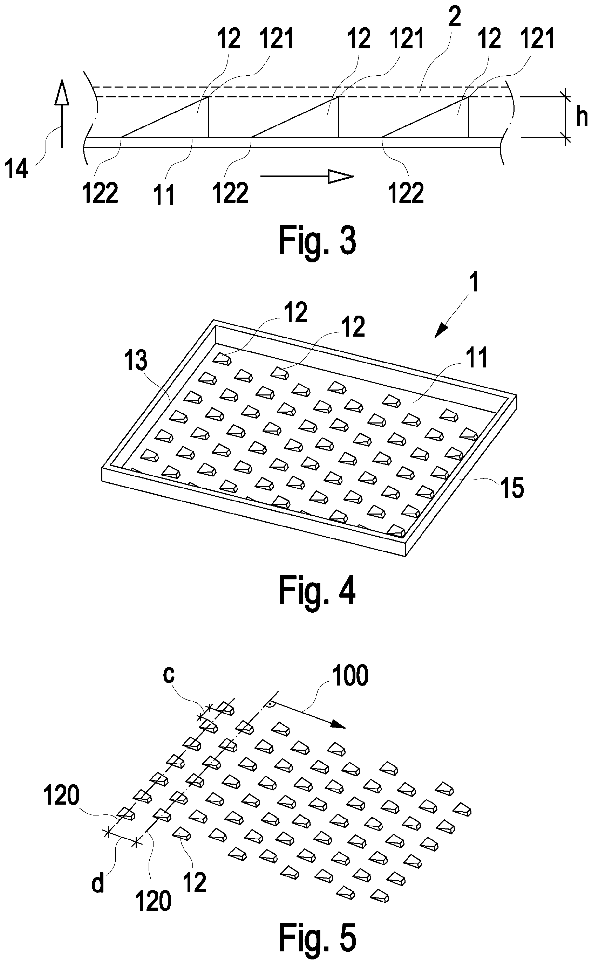

Fig. 3 shows a cut-through through an exemplary embodiment of a flow control tray according to the current invention wherein it can be seen that a plurality of elevations 12 is formed within the control tray which extend from a ground layer 11 which is formed in the embodiment according to Fig. 3 with equal height and the plurality of elevations 12 extend from the aforementioned ground layer 11 in a first direction 14. The plurality of elevations 12 extends from a triangular base 122 in the ground plane 11 to the support 121 which is distanced in the first direction 14 to the ground plane. The upper supports 121 are arranged in a first support height h above/distanced to the ground plane 11. In Fig. 3, furthermore, the algae cultivation membrane 2 is shown and supported by the plurality of supports 121 of the elevations 12.

In Fig. 4, a further exemplary embodiment of a flow control tray 1 is shown wherein the ground layer 11 is formed with side walls to form a tub-shaped ground layer 11.

In Fig. 5, a plurality of elevations 12 are shown wherein the elevations 12 are arranged in a plurality of lines 120, wherein the lines 120 extend orthogonal to the general flow direction 100, wherein the lines 120 are evenly distributed along the general flow direction 100 and spaced apart by a row line distance D. The plurality of elevations 12 arranged in a line 120 are spaced apart with respect to each other in the direction of the line 120 by a clearance distance C. As can be taken from Fig. 5, the line patterns of the plurality of elevations 12 of two consecutive lines 120 seen in the general flow direction 100 are offset with respect to each other to form an alternating structure of the plurality of elevations 12. The line patterns are offset in a direction orthogonal to the general flow direction 100.

## Claims

1. Flow control tray (1) for an algae cultivation fluid comprising:
a ground layer (11) extending in a ground plane from a first end (13) to a second end (15); and
a plurality of elevations (12) extending from the ground layer (11) in a first direction (14),
wherein the ground layer (11) is adapted to guide a flow of an algae cultivation liquid above the ground plane from the first end (13) to the second end (15) to form a general flow direction (100) in parallel to the ground layer,
wherein each of the plurality of elevations (12) comprises a support (121) distanced in a direction orthogonally to the ground plane and adapted to support an algae cultivation membrane (2), and
wherein each of the plurality of elevations (12) has a three-dimensional shape preferably adapted to divert the cultivation fluid from the general flow direction (100).

2. Tray (1) according to claim 1, wherein each of the plurality of elevations (12) has a three-dimensional shape to induce a laminar vortex with a horizontal axis of rotation in the cultivation fluid.

3. Tray (1) according to claim 1 or 2, wherein each support (121) of the plurality of elevations (12) is arranged in a second supporting plane parallel to the ground plane.

4. Tray (1) according to any one of the preceding claims, wherein each of the elevations (12) is formed as a ramp extending from the ground plane (11) in the first direction (14) to the upper support (121) in a first support height (h).

5. Tray (1) according to any one of the preceding claims, wherein the support (121) of each elevation (12) is formed by:
an upper support surface (121C),
an upper support edge (121B), or
an upper support point (121A).

6. Tray (1) according to any one of the preceding claims, wherein the elevations (12) are formed as a triangular ramp extending in the direction of the general flow direction (100) from a triangular base (122) in the ground plane to the support (121) distanced in the first direction (14) to the ground plane, wherein a width of the triangular ramp narrows in the general flow direction (100).

7. Tray (1) according to claim 6, wherein the triangular ramps are provided with a triangular tip (121A) as support (121) for the membrane (2) or wherein the triangular ramps are formed as truncated triangular with a triangular edge (121B) as support (121) for the membrane (2).

8. Tray (1) according to any one of the preceding claims, wherein the elevations (12) are evenly distributed over an area of the ground layer (11).

9. Tray (1) according to any one of the preceding claims, wherein the elevations (12) are arranged in a plurality of lines (120), wherein the lines (120) extend orthogonal to the general flow direction (100), wherein the lines (120) are evenly distributed along the general flow direction (100) and spaced apart by a row distance (d).

10. Tray (1) according to claim 9, wherein the elevations (12) arranged in a line (120) are spaced apart with respect to each other in the direction of the line (120) by a clearance distance (c).

11. System for algae cultivation comprising:
- a tray (1) according to any one of claims 1 to 10; and
- an algae cultivation layer (2).

12. System according to claim 11, wherein the algae cultivation layer (2) is formed by a fluid permeable membrane.

13. System according to claim 11 or 12, wherein the algae cultivation layer (2) is formed by a hydrophilic cultivation membrane with a pore size below 50 µm.

14. System according to any one of claims 11 to 13, wherein the algae cultivation layer (2) is formed by an algae cultivation membrane with a thickness of 50 µm and/or with a pore size of less than 50 µm preferably formed by polybutylene terephthalate.

15. System according to any one of claims 11 to 14, wherein the system further comprises a liquid supply unit which is configured to be arranged in the area of the first end (13) of the ground layer (11) and further configured to evenly supply the algae cultivation fluid to the first end (13) of the ground layer (11).

16. Usage of a system for algae cultivation according to any one of claims 11 to 15 for the cultivation of microalgae, especially for the cultivation of Astaxanthin-containing microalgae.
